Europäisches Patentamt

European Patent Office

Office européen des brevets

(11) Veröffentlichungsnummer: **0 255 145**
**A2**

(12) **EUROPÄISCHE PATENTANMELDUNG**

(21) Anmeldenummer: 87111117.5

(22) Anmeldetag: 31.07.87

(51) Int. Cl.4 **A61K 33/40** , A61L 2/00

(30) Priorität: 31.07.86 DE 3625867

(43) Veröffentlichungstag der Anmeldung:
03.02.88 Patentblatt 88/05

(84) Benannte Vertragsstaaten:
AT BE CH ES FR GB IT LI NL SE

(71) Anmelder: OXO-Chemie GmbH
Maassstrasse 24
D-6900 Heidelberg(DE)

(72) Erfinder: Elstner, Erich, Prof. Dr.
Wildmoosstrasse 28
D-8031 Gröbenzell(DE)
Erfinder: Kühne, Friedrich Wilhelm, Dr.
Bergstrasse 72
D-6900 Heidelberg(DE)
Erfinder: Adamczyk, Rainer, Dr.
Waldstrasse 45
D-8011 Brunnthal(DE)

(74) Vertreter: Kolb, Helga, Dr. Dipl.-Chem. et al
Hoffmann, Eitle & Partner Patentanwälte
Arabellastrasse 4
D-8000 München 81(DE)

(54) Verwendung von Tetrachlordecaoxid in der Ophthalmologie.

(57) Es wird die Verwendung von Tetrachlordecaoxid zur Herstellung eines Ophthalmikums beschrieben, welches insbesondere zur Behandlung von bakteriellen und/oder abakteriellen Bindehauteffekten und zur Behandlung der Cornea geeignet ist sowie zur Herstellung eines Reinigungs-und Aufbewahrungsmittels von Kontaktlinsen, wobei Tetrachlordecaoxid als Depot-$O_2$-Träger wirkt.

EP 0 255 145 A2

## Verwendung von Tetrachlordecaoxid in der Ophthalmologie

Die Erfindung betrifft die Verwendung von Tetrachlordecaoxid (TCDO) in der Ophthalmologie, insbesondere die Verwendung von TCDO als Ophthalmikum, insbesondere zur Behandlung von bakteriellen und/oder abakteriellen Bindehautaffekten, zur Behandlung der Hornhaut, sowie als Reinigungs-und/oder Aufbewahrungsmittel für Kontaktlinsen und als Depot-$O_2$-Träger.

Es ist in der Medizin bereits bekannt, zur schnelleren Wundheilung Sauerstoffträger zu verwenden. In P 34 38 966 wird eine Zubereitung zur Freisetzung von aktiviertem Sauerstoff und deren Verwendung beschrieben. Diese Zubereitung enthält stabilisierte Chloritmatrices in Lösung, wobei aus dieser Lösung durch Zugabe einer Fe-Porphyrin-Verbindung molekularer Sauerstoff freigesetzt wird.

Die allgemeine Formel dieser stabilisierten Chloritmatrix kann wie folgt beschrieben werden: $ClO_2^- .nO_2$, wobei n die Werte von 0.01 bis 0,25 annehmen kann. Tetrachlordecaoxid kann zur Deckung des biochemischen Sauerstoffbedarfes dienen. Die Anwendung von TCDO zur bakteriziden Wundreinigung ist bekannt. Es hat sich gezeigt, daß der neuartige Sauerstoffkomplex insbesondere unter Einwirkung von UV-B-Strahlung (300 bis 380 nm) in die physiologisch unbedenklichen Metabolite Sauerstoff und Chlorid abgebaut wird; dabei entsteht u.a. aktivierter Sauerstoff, der bakterizid und fungizid wirkt. Diese Anwendung von TCDO ist u.a. in der deutschen Patentanmeldung P 32 13 389.8 beschrieben.

Die Hornhaut (Cornea) von Kontaktlinsenträgern zeigt häufig Neovaskularisation als Folge eines Sauerstoffmangels. Dieser Sauerstoffmangel tritt auf, wenn durch die Kontaktlinse die Sauerstoffdiffusion behindert ist.

Außerdem ist es beim Tragen von Kontaktlinsen von besonderer Bedeutung, daß dieselben frei von Oberflächenablagerungen sind, um den Tragekomfort und die optimale Sehkraft zu gewährleisten. Es ist bekannt, daß Kontaktlinsen auf ihrer Oberfläche Ablagerungen von äußeren Quellen sowie von endogenen Quellen ansammeln. Dabei ist es schwierig, diese Verunreinigungen von den Kontaktlinsen vollständig zu entfernen, ohne dabei diese zu beschädigen. In den bisher bekannten Reinigungslösungen für harte und weiche Kontaktlinsen sind eine Vielzahl von wasserlöslichen Reinigungsmitteln zusätzlich zu wasserlöslichen hydratisierten Polymeren in sterilen, homogenen wäßrigen Lösungen verwendet worden. In DE-OS 30 21 034 wird ein Reinigungsmittel für Kontaktlinsen beschrieben, das ein oberflächenaktives Mittel, ein anorganisches Schleifmittel, ein Trägermaterial sowie gegebenenfalls ein Suspendiermittel umfaßt.

Da den zu reinigenden Kontaktlinsen auch Mikroorganismen anhaften können, besteht ein Bedarf nach einem Reinigungsmittel für Kontaktlinsen, das - über den üblichen Reinigungseffekt hinaus - bakterizid und fungizid wirkt, und diese Eigenschaft auch beim Tragen der Kontaktlinse über einen längeren Zeitraum beibehält.

Im weiteren ist bekannt, daß weiche Kontaktlinsen aufgrund ihrer größeren Porosität und Sauerstoffdurchlässigkeit für den Linsenträger vorteilhafter sind. Dennoch besteht auch beim Tragen poröser Kontaktlinsen die Gefahr einer Ausbildung von Anoxie aufgrund einer mangelnden Sauerstoffversorgung der Cornea. Um eine ausreichende Sauerstoffzufuhr im Hornhautbereich zu gewährleisten, besteht ein Bedarf nach in geeigneter Weise strukturierten und präparierten Kontaktlinsen.

Die Aufgabe der vorliegenden Erfindung besteht in der zur Verfügungstellung eines Ophthalmikums, das u.a. besonders geeignet ist zur Behandlung von bakteriellen und/oder abakteriellen Bindehautaffekten, zur Behandlung des Auges (Cornea) sowie in der Schaffung eines Reinigungs-und Aufbewahrungsmittels für Kontaktlinsen, wobei das Mittel in Kombination mit einer bakteriziden und fungiziden Wirkung vorzugsweise auch eine langsame Freisetzung von Sauerstoff gewährleistet.

Die vorstehende Aufgabe wird gelöst durch die Verwendung von Tetrachlordecaoxid zur Herstellung eines Ophthalmikums.

Die Ophthalmika eignen sich zur Prophylaxe und Behandlung von Entzündungen, wobei durch die erhöhte Versorgung des Auges mit Sauerstoff gleichzeitig eine bestehende Anoxie behoben bzw. einer solchen vorgebeugt wird. Es hat sich gezeigt, daß TCDO einen potentiellen Sauerstoffträger und -spender bei allen Sauerstoffproblemen im Augenbereich darstellt und sich z.B. zur Behandlung von Vaskularsektionen nach Verätzungen, Verbrennungen, Verletzungen und bei HM-Übertragungen eignet.

Im weiteren umfaßt die Erfindung die Verwendung von Tetrachlordecaoxid zur Herstellung eines Arzneimittels zur Behandlung von bakteriellen und/oder abakteriellen Bindehautaffekten und zur Behandlung der Cornea des Auges.

Darüber hinaus schließt die Erfindung auch die Verwendung von Tetrachlordecaoxid als Reinigungs-und/oder Aufbewahrungsmittel von Kontaktlinsen ein, wobei TCDO neben seiner anti-bakteriellen Wirkung als Depot-$O_2$-Träger in den Kontaktlinsen dient.

Die Konzentration an TCDO in den Ophthalmika liegt zwischen 10 und 25 mM, vorzugsweise beträgt sie 15 mM. Die Konzentration in dem Reinigungs-bzw. Aufbewahrungsmittel für Kontaktlinsen kann über diesem Bereich liegen, d.h. diese Konzentration kann von 10 bis 50 mM gehen. Es konnte am Kaninchen gezeigt werden, daß in den Augentropfen etc. eine Konzentration an TCDO von vorzugsweise 15 mM weder irritierend noch entzündend wirkt.

Das Ophthalmikum gemäß der Erfindung liegt vorzugsweise als wäßrige oder gepufferte Lösung vor. Dies trifft auch für das Reinigungs-oder Aufbewahrungsmittel für Kontaktlinsen zu. Während für das Ophthalmikum in Form von Augentropfen etc. die auf diesem Gebiet üblichen Pufferlösungen und Zusätze verwendet werden, eignen sich für das Reinigungs-und Aufbewahrungsmittel als Puffer z.B. Borsäure/Natriumborat, Phosphorsäure/Dinatriumphosphat und Natriumbicarbonat. Das Reinigungsmittel weist im allgemeinen einen pH-Bereich von 5 bis 8, vorzugsweise von 7 bis 8 auf.

Im übrigen kann das Reinigungsmittel übliche Hilfsstoffe, die auf das Auge nicht irritierend wirken, enthalten.

Darüber hinaus ist es auch möglich, das Reinigungsmittel für Kontaktlinsen in Form einer halbviskosen Paste zu formulieren. Ein solches Reinigungsmittel eignet sich für härtere Kontaktlinsen.

Sofern das Reinigungsmittel für Kontaktlinsen eine gesteigerte Reinigungswirkung besitzen soll, und das genannte Reinigungsmittel nach der Einlagerung der Kontaktlinsen vor Applikation auf dem Auge nicht abgespült wird, kann das Reinigungsmittel ein geeignetes oberflächenaktives Mittel, gegebenenfalls ein Schleifmittel und Suspendiermittel für dasselbe enthalten.

Eine Formulierung des Reinigungsmittels sieht die Zugabe eines oberflächenaktiven Mittels vor. Oberflächenaktive Mittel werden in Konzentrationen von 0,1 bis 40 Gew.%, vorzugsweise 0,5 bis 3 %, zugegeben. Beispiele für geeignete oberflächenaktive Mittel sind Natriumcetylsulfat, Natriumlaurylsulfat, Natriumoctylsulfat, Natriumdodecylsulfat, Natriumoleylsulfat, Ammoniumlauryethersulfat und Magnesiumlaurylethersulfat.

Als Schleifmittel eignen sich wasserunlösliche, anorganische Verbindungen. Bevorzugte Schleifmittel sind Siliciumdioxid, Aluminiumtrioxid, Manganoxid, Zirkonoxid, Kaolin, und die entsprechenden Carbonate sowie Calciumcarbonat.

Die Konzentration des Schleifmittels beträgt vorzugsweise 0,1 bis 25 Gew.%. Die Teilchengröße des Schleifmittels, die normalerweise angewendet wird, ist vergleichbar mit der oder etwas kleiner als die von Schleifmitteln, wie sie zum Polieren von Linsenoberflächen während deren Herstellung verwendet werden. Diese Teilchen sollen gleichmäßig in der Lösung des Reinigungsmittels suspendiert bleiben, ohne sich abzusetzen, was durch Zugabe eines Suspendiermittels erzielt wird. Vorzugsweise verwendet man Schleifmaterialien mit durchschnittlichen Teilchengrößen von 10 μm oder weniger.

Wie vorstehend erwähnt, werden die dem Reinigungsmittel zugegebenen Schleifmittel mit Hilfe eines Suspendiermittels in Suspension gehalten. Eine solche Suspension wird durch Erhöhung der Viskosität der wäßrigen Lösungen mit Hilfe von löslichen Salzen oder hydrophilen Polymeren erzielt. Geeignet sind dabei auch wasserlösliche, neutrale oder ionische Polymere, die mit der Oberfläche des Schleifmittels zusammenwirken können und dadurch dessen Ausfällen verhindern.

Als Suspendiermittel eignen sich insbesondere: Alkalihalogenide, Erdalkalisalze, Polyvinylalkohol, Polyacrylamid, Polyacrylsäure, Carboxymethylcellulose, Methylcellulose, Poly-N-vinylpyrrolidon, natürliches Gummi, wie Guargum, Tone, wie Bentonit, Montmorillonit sowie neutrale, kationische und anionische Detergenzien.

Die Reinigung der Kontaktlinsen erfolgt üblicherweise indem man die Linsen in die Reinigungslösung einlegt und - sofern die Formulierung des Reinigungsmittels dies vorsieht - daraufhin die Linsen unmittelbar auf das Auge aufsetzt. Durch das Einlegen der Linsen in wäßrige TCDO-Lösung nehmen insbesondere poröse Kontaktlinsen eine bestimmte Menge an TCDO auf. Versuche haben gezeigt, daß durch Lagern in 15 mM Tetrachlordecaoxid eine ausreichende Menge dieser Substanz von den Haftschalen aufgenommen wird, so daß dann in einer langsamen Aktivierungsreaktion von TCDO (durch Komponenten der Tränenflüssigkeit) eine bakterizide und fungizide Wirkung einsetzt, sowie gleichzeitig der Linsenbereich mit Sauerstoff versorgt wird. Diese Freisetzung wird insbesondere durch Einwirkung von UV-B-Strahlen, wie sie während des Tages auf das Auge einwirken, beschleunigt. Durch die Versorgung des Linsenbereiches mit Sauerstoff kommt es zu einer Verhinderung der Anoxie. TCDO wirkt auf die Augen nicht irritierend oder entzündungsauslösend.

Sofern die Formulierung des Reinigungsmittels eine gesteigerte Reinigungswirkung auf die Kontaktlinsen vorsieht, d.h. oberflächenaktive Mittel in höherer Konzentration, Schleifmittel und Suspendiermittel enthält, ist vorzugeben, nach Einlegen der Kontaktlinsen, z.B. über Nacht, dieselben vor Applikation auf das Auge mit Wasser zuspülen. In diesem Falle wirkt TCDO aufgrund seiner bakteriziden und fungiziden Eigenschaften beim Lagern in Kombination mit dem durch oberflächenaktive Mittel und Schleifmittel bewirkten Reinigungseffekt.

Beispiel 1

Eine Reinigungslösung, die bevorzugt für weiche Kontaktlinsen verwendet wird, und welche nach dem Einlegen der Linsen nicht abgespült wird, weist vorzugsweise die folgende Zusammensetzung auf:
TCDO      16 mM
destilliertes Wasser

Beispiel 2

Reinigungsmittel, wobei die Linsen nach dem Einlegen mit Wasser gespült werden:
Zusammensetzung:
TCDO      50 mM
destilliertes Wasser      200 g
Natriumdidecylethersulfat (30 %ig)      80 g
Natriumchlorid      20 g
Siliciumdioxid (8µm Durchschnittsteilchengröße)      20 g

Die folgenden Untersuchungen zeigen, daß Tetrachlordecaoxid als Sauerstoffträger in Kontaktlinsen aufgenommen, dort gespeichert und langsam wieder in die Tränenflüssigkeit bzw. in die Cornea abgegeben wird, um dort in die Metabolite Wasser, Chlorid und Sauerstoff zu zerfallen. Die Untersuchung hat gezeigt, welcher Kontaktlinsentyp zur Speicherung von TCDO besonders geeignet ist und mit welcher Geschwindigkeit Kontaktlinsen TCDO an ihre Umgebung freisetzen, um dort sowohl bakterizid zu wirken als auch den Sauerstoff-Partialdruck anzuheben.

Methoden und Ergebnisse

Verschiedene Kontaktlinsen wurden in TCDO-Lösungen von 16 mM TCDO über Nacht inkubiert. Die inkubierten Kontaktlinsen wurden anschließend mit destilliertem Wasser abgespült und nach Youngman et al (1985) mit Hämin und ACC in Pufferlösung 30 Min. inkubiert. Nach dieser 30-minütigen Inkubation wurde durch den gasdichten Gummistöpsel 1 ml Gas aus dem Kopfvolumen entnommen und gaschromatographisch analysiert. Die Menge des abgegebenen Ethylens ist proportional zu der Menge des gespeicherten TCDOs in dem jeweiligen Kontaktlinsentyp. Diese Versuchsbedingungen sind in Tabelle I angegeben.

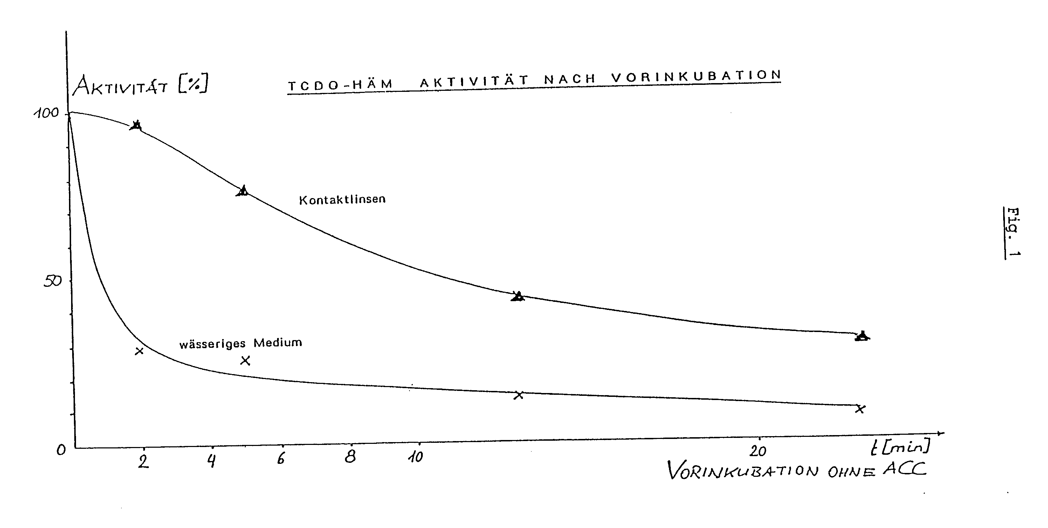

In Tabelle II sind verschiedene Kontaktlinsen parallel zueinander untersucht worden. Es wurde eingeteilt in harte Linsen und in weiche Linsen. Sowohl harte Linsen als auch weiche Linsen wurden unter identischen Bedingungen über Nacht in einer 16 mM TCDO-Lösung inkubiert. Nach der Inkubation wurden die Linsen herausgenommen und mit ACC und Hämin je 30 Min. inkubiert. Anhand einer Eichkurve wurde die gemessene Ethylenmenge auf die Menge TCDO bezogen. Nach dem Versuch wurden die Linsen über Nacht gegen Wasser dialysiert und die dialysierten Linsen getrocknet und erneut in TCDO-Lösung inkubiert und in einem zweiten Ansatz der Versuch wiederholt. Wie aus Tabelle II hervorgeht, sammeln harte Linsen relativ wenig TCDO. In einem halbstündigen Ansatz konnten jeweils zwischen 0,01 und 0,6 pMol TCDO pro Linsenmaterial aus dem Speicher freigesetzt werden. Im unteren Teil der Tabelle II sind die Werte für weiche Linsen wiedergegeben. Wie klar ersichtlich ist, wurde ein Vielfaches der TCDO-Menge in weichen Linsen gespeichert verglichen zu harten Linsen. Hier bewegen sich die Werte zwischen ungefähr 17 pMol TCDO pro 10 mg und 33 pMol TCDO pro 10 mg Linsengewicht. Weiche Linsen speichern somit mindestens das 100 bis 200fache an TCDO im Vergleich zu harten Linsen. Linsen eines Mitteltyps (nicht gezeigt) enthalten etwa 2 pMol TCDO pro 10 mg Linsengewicht. Der Freisetzungsprozeß von TCDO aus dem Linsenmaterial ist in Figur 1 wiedergegeben.

Aus früheren Experimenten war bekannt, daß TCDO mit einer bestimmten Häm-Menge schon nach 2 bis 5 Minuten zu mehr als 70 % abgebaut wurde. Dieses Ergebnis konnte bestätigt werden (Fig. I unterer Kurvenast). Im Gegensatz dazu entlassen inkubierte Kontaktlinsen nach 2 Minuten nur etwa 5 % der gespeicherten TCDO-Menge, die in das wäßrige Medium abgegeben und durch Häm zersetzt wurden. Hiermit ergibt sich durch den Speichereffekt der weichen Kontaktlinsen ein enormer Verzögerungseffekt der TCDO-Reaktion mit Häm. Die Gesamtreaktion wird von 2 Minuten auf etwa 20 Minuten ausgedehnt. Somit kann als bewiesen gelten, daß Tetrachlordecaoxid von weichen Kontaktlinsen aufgenommen, gespeichert und an das unmittelbare umgebenede wäßrige Medium in einem Depoteffekt abgegeben wird. Ein großer Teil des gespeicherten und abgegebenen TCDOs wird also die Cornea erreichen und dort den gewünschten Effekt auslösen.

Tabelle I

Inkübation von Kontaktlinsen (KL) in 16 mM TCDO (TCDO ist die Kurzbezeichnung für Tetrachlordecaoxid (Chlor(IV)-oxid-Sauerstoff-Komplex (4:1-Hydrat); das Produkt ist von Oxo-Chemie GmbH, Neckarsulm, erhältlich) und Nachweis der aufgenommenen Menge durch die TCDO/Hämin-getriebene Spaltung von Aminocyclopropancarbonsäure (ACC) Youngman et al (1985).

Ansatz zur ACC-Spaltung (Volumen: 2 ml) Hämin      50 uM
ACC      2,5 mM
0.2 M PO$_4$-Puffer, pH 7,8      1 ml
H$_2$O      ad 2 ml

Inkubationszeit von KI in TCDO:      12 Stunden
Inkubationszeit für Nachweisreaktion:      30 Minuten

Die ermittelten Werte beziehen sich auf 1 ml Reaktionsansatz.

Tabelle II

Häminaktivierte Ethylenfreisetzung aus ACC durch
TCDO-inkubierte Kontaktlinsen (KL)

| Linsentyp | pmol TCDO gespeichert / 10 mg KL | | |
|---|---|---|---|
| (Kontrollnummer) | 1. Ansatz | 2. Ansatz | |
| hart | | | |
| 1 | 0,070 | 0,540 | |
| 2 | 0,009 | 0,040 | |
| 3 | 0,022 | 0,027 | |
| 4 | 0,013 | 0,058 | |
| 5 | 0,390 | 0,250 | |
| 7 | 0,007 | 0,037 | |
| 9 | 0,000 | 0,057 | |
| 12 | 0,360 | 0,079 | |
| 13 | 0,074 | 0,059 | |
| 17 | 0,007 | 0,067 | n = 23 |
| 18 | 0,022 | 0,026 | $\bar{x}$ = 0,099 |
| 21 | 0,016 | 0,036 | x $\sigma$ n = 0,14 |
| weich | | | |
| 6 | 17,21 | 26,27 | |
| 8 | 19,65 | 24,02 | |
| 10 | 17,83 | 22,91 | |
| 11 | 24,34 | 29,33 | |
| 14 | 33,46 | 25,57 | |
| 15 | 20,05 | 28,94 | n = 16 |
| 16 | 18,92 | 24,91 | $\bar{x}$ = 23,44 |
| 19 | 19,34 | 22,23 | x $\sigma$ n = 4,45 |

**Ansprüche**

1. Verwendung von Tetrachlordecaoxid (TCDO) zur Herstellung eines Ophthalmikums.

2. Verwendung nach Anspruch 1 zur Herstellung eines Arzneimittels zur Behandlung von bakteriellen und/oder abakteriellen Bindehautaffekten

3. Verwendung nach Anspruch 1 zur Herstellung eines Arzneimittels zur Behandlung der Hornhaut des Auges.

4. Verwendung von Tetrachlordecaoxid zusammen mit einem geeigneten Trägermaterial als Reinigungsmittel bzw. Aufbewahrungsmittel von Kontaktlinsen

5. Verwendung von Tetrachlordecaoxid nach Anspruch 3 oder 4 als Depot O$_2$-Träger für Kontaktlinsen

6. Verwendung nach Anspruch 2 oder 3, wobei TCDO in einer Konzentration von 10 bis 25 mM, vorzugsweise von 15 mM, vorliegt.

7. Verwendung nach Anspruch 4 oder 5, wobei TCDO in einer Konzentration von 10 bis 50 mM vorliegt

8. Verwendung nach einem oder mehreren der vorhergehenden Ansprüche, wobei als Trägermaterial Wasser dient

9. Verwendung nach einem oder mehreren der vorhergehenden Ansprüche, wobei als Trägermaterial eine Pufferlösung dient.

10. Verwendung nach einem oder mehreren der vorhergehenden Ansprüche, wobei TCDO zusammen mit dem Trägermaterial in Form einer halbviskosen Paste vorliegt.

11. Verfahren nach Anspruch 4 oder 5, wobei das Mittel im weiteren ein oberflächenaktives Mittel enthält.

12. Verwendung nach Anspruch 11, wobei das oberflächenaktive Mittel in einer Menge von 1 bis 40 Gew.% vorliegt.

13. Verwendung nach Anspruch 7, wobei das Mittel im weiteren ein anorganisches Schleifmittel enthält.

14. Verwendung nach Anspruch 13, wobei das Schleifmittel in einer Konzentration von 0,1 bis 25 Gew.% vorliegt.

15. Verwendung nach Anspruch 13 oder 14, wobei das Schleifmittel aus der Gruppe Siliciumdioxid, Aluminiumdioxid, Kaolin, Calciumcarbonat, Zirkonoxid und Mischungen derselben ausgewählt ist.

16. Verwendung nach Anspruch 4 oder 5, wobei das Mittel im weiteren ein Suspendiermittel enthält.

17. Verwendung nach Anspruch 16, wobei das Suspendiermittel aus anorganischen Salzen und hydrophilen Polymeren ausgewählt ist.

Fig. 1

TCDO-HÄM AKTIVITÄT NACH VORINKUBATION

AKTIVITÄT [%]

100

50

0

Kontaktlinsen

wässeriges Medium

2   4   6   8   10   20   t [min]

VORINKUBATION OHNE ACC